# EUROPÄISCHE PATENTANMELDUNG

(11) **EP 1 022 554 A1**
(43) Veröffentlichungstag der Anmeldung: **26.07.2000**
(21) Anmeldenummer: 99810040.8
(22) Anmeldetag: 20.01.1999
(51) Int. Cl.: G01N 1/02, G01N 33/00, G01N 33/22, C07D 231/54, A61B 10/00

(54) **Vorrichtung zum Nachweis von chemischen Substanzen und Spuren, sowie Nachweisverfahren**

(71) Anmelder: Universität Bern Institut für Rechtsmedizin (IRM), 3012 Bern (CH)
(72) Erfinder: Bruhin, Thomas, 8853 Lachen (CH); Kilchör, 3013 Bern (CH)
(74) Vertreter: BOVARD AG - Patentanwälte

(57) **Zusammenfassung**

Die Vorrichtung und das Verfahren dienen zur Detektion von chemischen Substanzen und Spuren, insbesondere von Schmauchspuren nach Schusswaffengebrauch. Die Vorrichtung kann als klebstoffbeschichteter Rollstab ausgebildet sein. Er dient zum Abrollen über die zu prüfende Körperstelle oder den zu prüfenden Gegenstand. Die sicherzustellenden chemischen Substanzen und Spuren haften so auf dem Klebstoff, der sich auf der Rolle des Stabes befindet. Anschliessend wird der Rollstab in einen zur Vorrichtung gehörenden, verschliessbaren Behälter z.B. ein Reagenzglas getaucht, welcher mit einem Lösungsmittel gefüllt sein kann. Der Nachweis der gesuchten chemischen Substanzen und Spuren kann anschliessend direkt im Reagenzglas durch eine zusätzliche nasschemische Reaktion erfolgen.

Die Anwendung des Verfahrens wird durch einen feldtauglichen Kit erleichtert, welcher Anleitung, Rollstäbe, Reagenzgläser, Chemikalien in einer lagerfähigen Zusammensetzung sowie Klebeetiketten und Klebesiegel enthält.

## Beschreibung

Die Erfindung betrifft eine Vorrichtung zum Nachweis von chemischen Substanzen und Spuren, insbesondere von nitrithaltigen Schmauchspuren, an Körperteilen und an Gegenständen und ein entsprechendes Nachweisverfahren. Besonders gut ist die Erfindung geeignet für die Ermittlung und den Nachweis von nitrithaltigen Schmauchspuren an der Schusshand sowie an anderen Körperteilen von mutmasslichen Schützen nach dem Gebrauch von Schusswaffen.

Zum Nachweis und zur Ermittlung von Schmauchspuren auf Körperteilen und Gegenständen nach Schusswaffengebrauch werden heute zunächst eine erste Probe als Vortest und dann eine zweite Probe als Bestätigungsanalyse erhoben. Dabei ist der Vortest wegweisend für unmittelbare Entscheide in den Ermittlungsarbeiten. Die zweite Probe dient dann zur Überprüfung und Bestätigung des Vortests und wird heute praktisch ausschliesslich mit Hilfe von sog. REM-Tabs zum Abtupfen der Körperteile und Gegenstände vorgenommen, wobei die Tabs anschliessend im Labor unter dem Rasterelektronenmikroskop analysiert werden.

Zur Untersuchung von tatverdächtigen Personen nach Abgabe eines Schusses aus einer Schusswaffe sind bereits zahlreiche Vortests entwickelt worden. In der praktischen Anwendung erbringen die heute bekannten Verfahren jedoch zum Teil nur wenig befriedigende Ergebnisse.

Der heute gebräuchlichste Vortest ist der Nachweis von schwermetallhaltigen Schmauchspuren (Blei, Barium und Antimon) mit Hilfe eines in Weinsäure getauchten Filterpapiers. Dabei wird das Filterpapier fest auf die Hand eines mutmasslichen Schützen gedrückt, damit allfällige Schwermetallspuren auf dem Filterpapier haften bleiben. Unmittelbar danach muss das Filterpapier mit einem Heissluftgebläse getrocknet werden. Wird es nicht sofort getrocknet, können die schwermetallhaltigen Schmauchspuren kaum mehr sichtbar gemacht werden. Anschliessend wird das Filterpapier mit einer Rhodozinat-Lösung besprüht, um die Spuren sichtbar zu machen. Da die Rhodozinat-Lösung nur kurze Zeit haltbar ist, erfolgt das Besprühen und damit der Nachweis nicht vor Ort, sondern im Labor. Dadurch verstreicht unter Umständen wertvolle Zeit, bis ein Resultat ausgewertet werden kann. Des weiteren ist es bei einigen Typen von Schusswaffen recht schwierig, nach einer Schussabgabe mit der Rhodozinat-Methode den Nachweis von Schmauchspuren zu erbringen, obwohl nachweislich geschossen worden ist. Zudem können mit der Rhodozinat-Methode nur bleihaltige Munitionsarten nachgewiesen werden. Heute werden aber immer mehr bleifreie Munitionsarten verwendet, so dass in diesen Fällen mit der Rhodozinat-Methode Schmauchspuren methodenbedingt gar nicht mehr nachgewiesen werden können.

Es ist Aufgabe der vorliegenden Erfindung, eine Vorrichtung und ein Verfahren zur Verfügung zu stellen, die frei von den obenerwähnten Nachteilen sind, wobei ein zuverlässiger Nachweis vor Ort ermöglicht werden soll.

Gegenstand der vorliegenden Erfindung sind demzufolge die Vorrichtung gemäss der Definition im Anspruch 1 und das Verfahren zum Nachweis von Spuren chemischer Substanzen an Körperteilen und Gegenständen gemäss der Definition im Anspruch 8 bis 11.

Vorteilhaft ist die Vorrichtung gemäss der vorliegenden Erfindung als Rollstab ausgebildet, der mit einem Haftkleber beschichtet ist, wobei der Rollstab in einem dazugehörigen verschliessbaren Reagenzglas aufbewahrt und transportiert wird. Dadurch wird die Haftkleberschicht vor äusseren Einflüssen geschützt.

Da die heute bekannten Munitionsarten alle Nitrocellulose enthalten und da deshalb nach der Schussabgabe nitrithaltige Schmauchspuren zurückbleiben, wird zum Nachweis der Schmauchspuren in erster Linie nach Nitrit gesucht. Die nachstehenden Ausführungen beziehen sich deshalb stellvertretend für andere Nachweisverfahren, auf welche die Vorrichtung gemäss der vorliegenden Erfindung anwendbar ist, auf ein derartiges Beispiel.

Beim erfindungsgemässen Verfahren wird die zu prüfende Körperstelle oder der zu prüfende Gegenstand mit der beschriebenen Vorrichtung abgetastet bzw. abgerollt, damit gegebenenfalls vorhandene Spuren der sicherzustellenden Substanzen auf der Haftklebeschicht haften bleiben. Danach wird die Vorrichtung, z.B. der Rollstab, in ein weiteres zugehöriges, verschliessbares Reagenzglas getaucht, welches mit Aceton oder einem anderen Lösungsmittel gefüllt ist. Dadurch löst sich die Klebeschicht mit der sicherzustellenden Substanz vom Träger oder Rollstab, welche für die folgenden Schritte nicht mehr benötigt werden.

In einem nächsten Schritt wird ebenfalls vor Ort im Reagenzglas das Nitrit mittels einer Kalium-Hydroxid-Lösung von der Nitrocellulose abgespalten und dann mit Hilfe von Sulfanilamid und N-(1-Naphthyl)-ethylendiammonumdichlorid in Phosphorsäure durch Bildung einer Azo-Kupplung sichtbar gemacht. Das Auftreten einer violett-roten Färbung der Lösung zeigt das Vorhandensein von Nitrit und damit die Schmauchspuren an.

Danach wird das beschriftete Reagenzglas mit der Probe versiegelt und kann so für Beweiszwecke aufbewahrt werden.

Wird nach anderen chemischen Substanzen gesucht, so ist der chemische Nachweis bzw. die Sichtbarmachung mit angepassten chemischen Mitteln, aber grundsätzlich mit der gleichen Vorrichtung zu erbringen.

Als Haftklebstoffe kommen beispielsweise Klebstoffe auf Basis von Polyacryat, Polyester, Polychloropren, Polyisobuten, oder Polyvinylether oder Mischungen davon in Frage. Die Klebstoffschicht weist vorzugsweise eine Dicke von 8 bis 20 µm auf. Der Klebstoff kann aufgesprüht, aufgerakelt oder auf andere Weise aufgetragen sein. Gut geeignet sind auch handelsübliche Klebebänder z.B. der Firma Scotch®, die beiseitig beschichtet sind. Sie können zur Auftragung einer Haftklebstoffschicht auf einen Träger bzw. eine Walze aufgeklebt werden.

Damit das erfindungsgemässe Verfahren einfach und in reproduzierbarer Weise durchgeführt werden kann, wird hierzu eine standardisierte Vorrichtung in Form eines kompletten, feldtauglichen Kits mit Anleitung, mehreren klebstoffbeschichteten Rollstäben und mehreren verschliessbaren Reagenzgläsern sowie mit Etiketten und Klebesiegeln vorgeschlagen. Dadurch kann das Verfahren auch von nicht spezialisiertem Personal direkt vor Ort unter erschwerten Bedingungen angewendet werden.

Typische Merkmale und Vorteile des erfindungsgemässen Nachweisverfahrens sind folgende:
- Die Spuren werden auf einem mit Haftklebstoff beschichteten Träger, vorzugsweise auf einem klebstoffbeschichteten Rollstab fixiert.
- Die so fixierten Spuren werden vor Ort direkt in einem beiliegenden, verschliessbaren Reagenzglas gelöst.
- Der Nachweis der Schmauchspuren erfolgt dann ebenfalls vor Ort im Reagenzglas durch Sichtbarmachen des Nitrits, welches in allen heute bekannten Munitionsarten vorhanden ist.
- Die Probe mit den Spuren kann direkt im verschlossenen, beschrifteten und mit einem nicht ohne Beschädigung aufreissbaren Klebesiegel gelagert werden.
- Im Gegensatz zu den bisherigen Verfahren kann der Nachweis innert 3 bis 5 Minuten direkt vor Ort erfolgen.
- Zur standardisierten Anwendung des Verfahrens wird eine Vorrichtung verwendet, welche in Form eines handlichen Kits sämtliche notwendigen Bestandteile (Anleitung, mehrere klebstoffbeschichtete Rollstäbe, mehrere verschliessbare Reagenzgläser, Klebeetiketten, Klebesiegel sowie die Chemikalien) enthält.
- Dieser Kit ist aufgrund der Auswahl und Zusammensetzung der gewählten Chemikalien praktisch unbegrenzt lagerfähig.

Die entscheidenden Vorteile für den Nachweis von Schmauchspuren mittels des erfindungsgemässen Verfahrens, d.h. der Rollstab-Methode, gegenüber den bekannten Vortests und insbesondere gegenüber dem Vortest nach der Rhodozinat-Methode sind die folgenden: Erstens ist der Nachweis an Ort und Stelle (Tatort) und damit innert kürzester Zeit (ca. 3 Minuten) möglich. Zweitens ist dafür kein besonders geschultes Laborpersonal nötig. Drittens sind die Vorrichtung und das Verfahren gemäss der vorliegenden Erfindung bei allen heute gebräuchlichen Munitionsarten einsetzbar. Es spielt also keine Rolle mehr, ob mit einer bleifreien oder bleihaltigen Munition geschossen wurde. Viertens erlaubt die hohe Empfindlichkeit des Verfahrens den Nachweis von Schmauchspuren auch bei Waffentypen, die wenig Schmauch abgeben. Fünftens sind der Rollstab und das Reagenzglas sehr handlich und klein, und es müssen keine Apparaturen mitgeführt werden.

Nachfolgend wird die Erfindung anhand einer speziellen Ausführungsform gemäss den beilegenden Zeichnungen näher erläutert, ohne dass dadurch eine Einschränkung der im Anspruchsteil definierten Erfindungen beabsichtigt ist. Es zeigt:
Fig. 1 eine Seitenansicht einer Ausführungsform einer erfindungsgemässen Vorrichtung umfassend einen Rollstab, ein Reaktionsgefäss und einen Deckel;
Fig. 2 einen Rollstab;
Fig. 3 einen Griff eines Rollstabes gemäss Fig. 2;
Fig. 4 eine mit einer Haftklebstoffschicht versehene Walze;
Fig. 5 eine Drehachse für die Walze gemäss Fig. 4;
Fig. 6 ein Schutz- und Reaktionsgefäss für einen Rollstab gemäss Fig. 2.

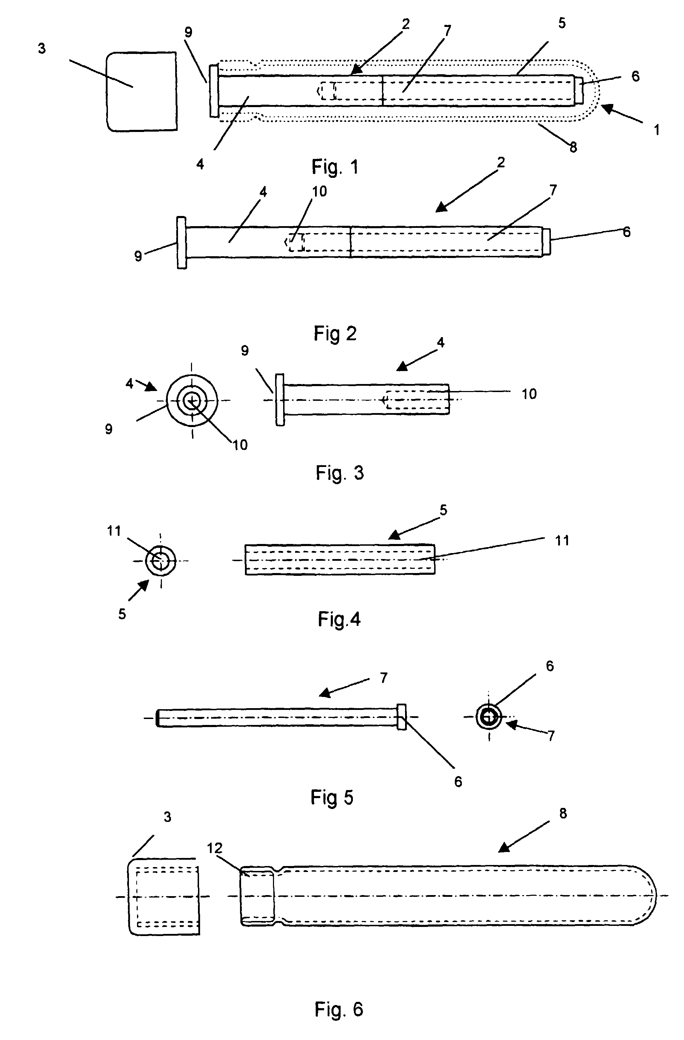

Fig. 1 zeigt eine Übersicht 1 eines Rollstabes 2 mit Aufbewahrungs- und Reaktionsgefäss 8 und passendem Deckel 3. Der Rollstab 2 besteht aus einem Griff 4, der zur Aufnahme einer Achse 7 bestimmt ist und der mit einer Dichtung 9 für das Reaktions- und Aufbewahrungsgefäss 8 versehen ist. Auf der Achse 7 ist die mit einer Haftklebstoffschicht versehene Walze 5 drehbar angeordnet. Am einen Ende der Achse ist ein Arretiermittel 6 vorgesehen, welches das Wegrutschen der Walze 5 verhindert. Das andere Ende der Achse ist so ausgestaltet, dass es für die Einführung und Fixierung im Griff geeignet ist.

Um unter Anwendung der in Fig. 1 dargestellten Vorrichtung Schmauchspuren zu asservieren, wird der Rollstab 2 aus dem verschliessbaren Reagenzglas 8 genommen, wobei der Rollstab 2 nur am Griff 4 angefasst werden darf. Darauf wird der drehbare Teil, d.h. die Walze 5, die eine Haftklebstoffbeschichtung aufweist, über die zu detektierende Fläche (Hände oder andere Körperteile, Gegenstände, Kleider oder überall, wo Schmauchspuren detektiert werden sollen) abgerollt. Dadurch werden die Spuren auf der Klebstoffschicht fixiert. Danach wird Aceton (ca. 0,5 ml) in das mitgelieferte Reagenzglas 8 gegeben. Anschliessend wird der Rollstab 2 in das Reagenzglas 8 eingetaucht. Durch leichtes Schwenken wird erreicht, dass das Aceton fixierte Substanzen von der Klebstoffschicht löst. Der Stab kann nun entfernt und allenfalls entsorgt werden, wobei die Acetonlösung im Reagenzglas zurückbleibt. Die weitere Detektion geschieht direkt im Reagenzglas vor Ort. Mit einer Kalium-Hydroxid-Lösung wird das Nitrit von der Nitrocellulose, welche in allen heute bekannten Munitionsarten vorhanden ist, abgespalten. Mit Hilfe einer Azokupplung kann dann das freie Nitrit sichtbar gemacht werden. Das Auftreten einer violett-roten Färbung der Lösung zeigt das Vorhandensein von Nitrit und damit die Schmauchspuren an. Damit kann festgestellt werden, wo im verschlossenen Reagenzglas Schmauchspuren anhaften. Danach kann die Probe aufbewahrt werden.

Ist der Nachweis positiv, erfolgt dann allenfalls eine zweite Probe als Bestätigungsanalyse z.B. mit der REM-Tab-Methode.

In Fig. 2 ist ein Rollstab 2 gezeigt, der zur Anwendung bereit ist, wobei die Bezugszeichen denjenigen von Fig. 1 entsprechen und 10 die Bohrung zeigt, welche für die Aufnahme der Achse 7 bestimmt ist.

Die Fig. 3, 4 und 5 zeigen Seitenansichten von den einzelnen Teilen eines Rollstabes gemäss der Erfindung, jeweils mit einer Ansicht der entsprechenden Stirnseiten.

Fig. 6 zeigt das Aufbewahrungs- und Reaktionsgefäss für einen Rollstab gemäss Fig. 7. Das Bezugszeichen 12 zeigt die Öffnung für die Einführung eines Rollstabes. Das verschliessbare Gefäss mit Deckel dient zur Lagerung und zum Transport des Rollstabs sowie zur Durchführung von chemischen Nachweisreaktionen. Das Gefäss dient auch zur Aufbewahrung der chemischen Substanzen, die für die jeweiligen Nachweisreaktionen erforderlich sind.

## Patentansprüche

1. Vorrichtung zur Aufnahme und zum Nachweis von Spuren von chemischen Substanzen an Körperteilen und Gegenständen, insbesondere für Schmauchspuren von Schusswaffen, gekennzeichnet durch einen mit einem Haltegriff versehenen Träger mit einer Oberfläche, die mindestens teilweise mit einem Haftklebstoff beschichtet ist, und einem Behälter, welcher zur Aufnahme des beschichteten Trägers bestimmt ist.

2. Vorrichtung nach Anspruch 1, dadurch gekennzeichnet, dass der mit einem Haltegriff versehene Träger eine Walze mit einem Haltegriff ist, an welchem die Walze axial drehbar befestigt ist, wobei die Walze auf ihrer zylindrischen Oberfläche eine Haftklebstoffbeschichtung aufweist.

3. Vorrichtung nach Anspruch 2, dadurch gekennzeichnet, dass sie als Rollstab ausgebildet ist, wobei am Haltegriff eine Achse fest oder abnehmbar angeordnet ist und die mit einem Haftklebstoff beschichtete Walze eine axiale Bohrung für die Aufnahme der Achse aufweist.

4. Vorrichtung nach einem der Ansprüche 1 bis 3, dadurch gekennzeichnet, dass der Behälter flüssigkeitsdicht verschliessbar ist, in solcher Weise, dass die Haftklebstoffbeschichtung des Trägers von äusseren Einflüssen geschützt ist und/oder mit einem sich im Behälter befindenden Reagens in Kontakt kommen kann.

5. Vorrichtung nach einem der Ansprüche 1 bis 4, dadurch gekennzeichnet, dass der Haltegriff des Trägers so ausgebildet ist, dass er in eingeführtem Zustand den Verschluss des Behälters bildet.

6. Vorrichtung nach einem der Ansprüche 1 bis 5, dadurch gekennzeichnet, dass der Behälter ein flüssiges Nachweisreagens enthält, welches bei der Anwendung der Vorrichtung einen direkten Nachweis von bestimmten durch die Haftklebstoffschicht aufgenommenen Spuren von chemischen Substanzen erlaubt.

7. Vorrichtung nach einem der Ansprüche 1 bis 6, dadurch gekennzeichnet, dass der Haftklebstoff aus Klebstoffen auf Basis von Polyacryat, Polyester, Polychloropren, Polyisobuten, oder Polyvinylether oder Mischungen davon ausgewählt ist und in einer Dicke von 8 bis 20 µm aufgetragen ist.

8. Verfahren zum Nachweis von Spuren von chemischen Substanzen an Körperteilen und Gegenständen, insbesondere von Schmauchspuren von Feuerwaffen, gekennzeichnet durch die nachstehenden aufeinanderfolgenden Verfahrensschritte
- Bereitstellen eines Abschnittes aus Selbstklebefolie, bestehend aus einer flexiblen Materialbahn, welche an der einen Seite mit einem Haftkleber beschichtet ist und auf der andern Seite unbeschichtet ist,
- Andrücken des Abschnittes aus Selbstklebefolie mit der beschichteten Seite auf einer Stelle am zu untersuchender Körperteil oder am Gegenstand, an welchen Spuren von chemischen Substanzen vermutet werden, indem Druck auf die unbeschichtete Seite ausgeübt wird,
- Entfernen des Selbstklebefolie und
- Überführen der Selbstklebefolie in einen Behälter und Verschliessen desselben.

9. Verfahren zum Nachweis von Spuren von chemischen Substanzen an Körperteilen und Gegenständen, insbesondere von Schmauchspuren von Feuerwaffen, gekennzeichnet durch die nachstehenden aufeinanderfolgenden Verfahrensschritte
- Bereitstellen eines Trägers mit einer Walze, welche auf ihrer Oberfläche mit einem Haftkleber beschichtet ist, so dass der Haftkleber frei zugänglich ist,
- Andrücken und Abrollen der Walze mit der beschichteten Seite auf eine Stelle am zu untersuchender Körperteil oder Gegenstand, an welchen Spuren von chemischen Substanzen vermutet werden,
- Überführen der Walze in einen Behälter und verschliessen desselben.

10. Verfahren nach Anspruch 8 oder 9, dadurch gekennzeichnet, dass der Behälter ein Nachweisreagens für die nachzuweisende Substanz enthält.

11. Verfahren nach Anspruch 10, dadurch gekennzeichnet, dass die nachzuweisende Substanz Nitrit ist und das Reagens Sulfanilamid und N-(1-Naphthyl)-ethylendiammonumdichlorid ist, wobei im positiven Fall ein roter Azofarbstoff erhalten wird.

12. Reagenziensatz zum Nachweis von Spuren von chemischen Substanzen an Körperteilen und Gegenständen, insbesondere von Schmauchspuren von Feuerwaffen, dadurch gekennzeichnet dass mindestens eine Vorrichtung nach einem der Ansprüche 1 bis 7 mit mindestens einem Nachweisreagens kombiniert ist.

13. Reagenziensatz zum Nachweis von Spuren von chemischen Substanzen an Körperteilen und Gegenständen, insbesondere von Schmauchspuren von Feuerwaffen, dadurch gekennzeichnet dass er Abschnitte aus Selbstklebefolie, bestehend aus einer flexiblen Materialbahn, welche an der einen Seite mit einem Haftkleber beschichtet ist und auf der anderen Seite unbeschichtet ist, Aufbewahrungs- oder Reaktionsbehälter und mindestens ein Nachweisreagens umfasst.
